# EUROPEAN PATENT APPLICATION

(11) **EP 1 990 048 A2**
(43) Date of publication of application: **12.11.2008**
(21) Application number: 08155677.1
(22) Date of filing: 06.05.2008
(51) Int. Cl.: A61K 31/137, A61K 31/167, A61K 31/194, A61K 31/195, A61K 31/277, A61K 31/42, A61K 31/421, A61K 31/4704, A61K 31/4706, A61K 31/4985, A61K 31/519, A61K 31/52, A61K 31/57, A61K 31/573, A61K 31/635, A61K 31/65, A61K 31/69, A61K 33/24, A61K 38/10, A61K 38/13, A61K 38/16

(54) **Therapy of benign prostatic hyperplasia (bph)**

(30) Priority: 08.05.2007 EP 07107700
(71) Applicant: Wiebelitz, Ulrike, 13158 Berlin (DE)
(72) Inventor: Wiebelitz, Ulrike, 13158 Berlin (DE)
(74) Representative: Vossius & Partner

(57) **Abstract**

The present invention relates to the use of immunomodulatory and/or immunosuppressive substances for the therapy of benign prostate hyperplasia (BPH) and non-bacterial prostatitis.

## Description

### Introduction

The present invention relates to the use of immunomodulatory and/or immunosuppressive substances for the therapy of benign prostatic hyperplasia (BPH) and non-bacterial prostatitis.

### State of the art

The prostate is a glandular organ of the male reproductive tract. The prostate is located anterior to the rectum, directly inferior to the bladder and surrounds the urethra. The prostate produces a secretion and supports ejaculation.

The prostate is composed of four zones. Approximately one third of the prostate is the anterior or fibro-muscular zone; the other parts are referred to as peripheral zone, central zone and transition zone.

The prostate grows continuously from a certain age on. This process can be detected in almost all men. The first phase of prostate growth occurs around the age of 25 and is related to the active reproduction phase. The second phase of growth sets in around the age of 50. This phase of growth results from changes in the hormonal balance of elder men. The decrease of the testosterone level and a relative dominance of estrogens result in a growth stimulus of the prostate tissue. The growth of the protaste is however limited by an outer capsule. This limitation by the capsule leads to an increased tissue pressure and a compression of the urethra enclosed by the prostate tissue. The disease is referred to as benign prostate hyperplasia (BPH) and is almost always accompanied by a non-bacterial prostatitis. The occurrence of BPH leads to an obstruction of the flow of urine due to compression. Due to the increased pressure in the bladder, small quantities of urine already cause bladder spasms which can be painful. The disturbed physiologic processes involve frequent urination which can cause pain to the patient. In the further progress of the disease, retention of urine occurs, since the bladder is no longer capable to empty completely. When BPH is first diagnosed, the patient reports on characteristic symptoms such as weak, intermittent urinary stream, post-void dribbling, frequent urination, in particular during sleep at night. Ultrasonography allows to show that the bladder is no longer emptied completely and that a certain amount of residual urine remains in the bladder.

BPH is a disease of elder men. Due to the continuously increasing life expectancy, the number of men suffering from BPH will increase more than proportionally. It has been estimated that about 80% of men above the age of 80 will suffer from a symptomatic BPH. In the United States alone, BPH is the reason for almost 400.000 hospitalisations per year. This high occurrence of the disease and the increased occurrence with elder people lead to new requirements with respect to therapy.

The mechanism of BPH has not yet been completely elucidated. In many cases, male sex hormones play a central role. The testosterone level of elder men decreases to about 1-2% in comparison to that of young men. The DHT (dihydrotestosterone) level, however, does not change. DHT is produced by 5-alpha-reductase from testosterone. DHT can stimulate the growth of prostate cells. A second important aspect is the relative preponderance of female sex hormones which are also capable of stimulating the growth of prostrate cells.

There are various options for therapy for patients suffering from a symptomatic BPH. With a mild form of BPH, the observation of the symptoms found (watchful waiting) prevails. This therapy option is chosen in the case of about 80% of patients at this stage of BPH. However, watchful waiting plays a minor role with patients showing moderate and serious symptoms. In most of these cases, physician and patient decide in favour of an invasive therapy. Methods such as TURP or balloon dilatation are applied. TURP refers to a method whereby, starting from the urethra, the prostate is resected by means of an instrument (transurethral resection of the prostate). By removing the proliferating prostate tissue, the compression of the urethra is relieved. The same therapeutic effect is achieved with balloon dilatation. In comparison with TURP, this treatment involves a high recidivity rate requiring repeated treatment. Both methods of treatment are invasive and cause heavy strain for the patient. TURP, in particular, has a high morbidity rate, with the prime factors being haemorrhages and traumata. In some cases, it is necessary that patients are hospitalised for long periods of time, causing high costs. In the further course, TURP-treated patients suffer from urethra strictures which also have to be treated. The most serious side effects of TURP are impotence and erectile dysfunction caused by damage to sensitive nerve threads. This has been the reason for intensive research into additional and enhanced therapy methods.

The medication therapies available at present affect the disease in different ways but do not fulfil expectations. Thus, a satisfactory improvement of the symptoms and the functional impairments has not yet been achieved. The most important substances for therapy are: finasteride (Proscar), dutasteride (Avodart), alfuzosine (Xatral), terazosine (Hydtrin), doxazosin (Cardura) and tamsulosin (Flomax). Of the substances mentioned, finasteride and dutasteride directly influence testosterone metabolism by inhibiting 5-alpha-reductase. This enzyme inhibition leads to a decrease of the DHT level. All other substances influence the tonicity of the smooth muscles and are part of the group of alpha-blockers. The relaxation of the smooth muscles of the urethra and the neck of the urinary bladder is to improve the flow of urine and counteract the obstruction of the flow of urine. There is hope that this therapy can stop further reactive growth of the prostrate by avoiding spasms and continued contractions. The administration of alpha-blockers, however, involves side effects. Since substances of this group of active agents affect also the tonicity of all other smooth muscles, they may cause hypotension, atony, dry mucous membranes, and disturbed ejaculation. The active agent group of the 5-alpha-reductase inhibitors also involve medically relevant side effects such as sexual dysfunction and gynecomastia. Moreover, the efficacy of the pharmaceuticals decreases by-and-by and, thus, the initial therapeutic effect is lost. It is possible to increase the limited effect of both groups of active agent by combining alpha-blockers and 5-alpha-reductase inhibitors. This combination therapy can be more effective than a mono-therapy, but, in many cases, it will only postpone causal surgical treatment. Additionally, the combination involves a clear increase of side effects, since the side effects of a therapy with 5-alpha-reductase inhibitors are added to those of a therapy with alpha-blockers.

The fact that medication therapies so far fail, in particular, to reduce the enlargement of the prostate and, thus, to remedy the voiding dysfunctions necessitates, in the case of most patients, the mechanical removal of the enlarged prostate tissue by the so-called ablative method. The physician can chose from different methods, such as the transurethral resection by means of rotating micro-knives, methods using thermal laser light or high-frequency ultrasound. The principle common to these methods is the reduction of the prostate volume. However, all these methods have drastic and serious side effects. These side effects are caused by the destruction of tissue which results in injuries to the patient concerned, which are often long-term. The patient's quality of life is affected by painful strictures, loss of libido and erectile dysfunctions. With view to these facts, there is need for a medication therapy which, on the one hand, is easy and safe to apply and, on the other hand, treats the causes of the disease. The pharmaceuticals available at present, however, do not meet these requirements.

In order to enhance the therapeutic effect and alleviate the severity of the accompanying side effects at the same time, new therapeutic approaches are sought. The combination of alpha-blockers with antiphlogistic substances is one possibility. Patent No. WO 2004/089379 documents the combination of the alpha-blocker tamsulosin with various antiphlogistic substances such as acetylsalicylic acid, mesalazin, ibuprofen, naproxen, flurbiprafen, fenoprofen, fenbufen, ketoprofen, indoprofen, priprofen, etc. Among the listed substance combinations, also the combination of tamsulosin with the antiphlogistic substance leflunomide (Arava) is mentioned.

### Description of the invention

Surprisingly, it was found that immunomodulatory and immunosuppressive substances as mono-substances are appropriate for treating BPH and/or non-bacterial prostatitis.

This is an unknown approach of causal therapy of BPH which is based on the therapeutic application of a mono-therapy with the above-mentioned substances.

Mono-substance according to the invention means that the pharmaceutical preparation does not contain significant amounts of other active agents and that no other active agents are administered at the same time as the active agents of the invention. Within the meaning of the present invention, this implies in particular that the immunomodulatory and immunosuppressive substances are not administered in combination with alpha-blockers. Nor are they administered in combination with 5-alpha-reductase inhibitors.

Members of the group of immunomodulatory and immunosuppressive substances can be selected from the group consisting of: adalimumab, azathioprine, chloroquine and hydroxychloroquine (anti-malaria agents), cyclosporine (cyclosporine A), D-penicillamine, etanercept, gold salts (e.g. sodium aurothiomalate, auranofine), infliximab, leflunomide, methotrexate (MTX), minocycline (a tretracycline antibiotic), sulfasalazine (SSZ), FK 506, glucocorticoids, SEGRA inhibitors, fingolimod (FTY-720), laquinimod, teriflunomid (HMR-1726), MBP-8298, daclizumab, MLN-1202, TV-5010, BG-12, ZK 117137, temsirolimus, sirolimus, tacrolimus (mitoxantron), basaliximab, MBP-8298, MLN-3897, CCX-915, MK0812, AZD-9056, BMS-345541 and bortezomib.

Leflunomide is most preferred as mono-substance for the treatment of BPH and/or non-bacterial prostatitis.

Leflunomide is a clinically applied pharmaceutical for the treatment of rheumatoid arthritis. By treatment with leflunomide, the progress of rheumatoid arthritis can be inhibited. In addition to the application of leflunomide, it is known that leflunomide can be used in combination with an alpha-blocker in the therapy of BPH. The combination of these different active agents is expected to increase the therapeutic affect of alpha-blockers. It is not yet known, however, that administration of leflunomide alone can treat BPH in a patient.

As described above, leflunomide is a pharmaceutical applied for the treatment of rheumathoid arthritis. In the present context it is administered in a single dose of 20 to 25 mg per day over a long period of therapy.

Moreover, the present invention relates to a pharmaceutical composition comprising immunomodulatory/immunosuppressive active agents as mono-substance for the treatment of BPH and/or non-bacterial prostatitis. A pharmaceutical composition comprising leflunomide as mono-substance is particularly preferred.

The therapeutic effect of the active agents when administered alone, in particular of leflunomide, on existing BPH has hitherto not been known. It was found that, in contrast to the already known combination of tamsulosin and leflunomide, the mono-therapy with leflunomide, in particular, leads to a fundamental amelioration of BPH. This therapeutic effect comprised both the amelioration of symptoms in a patient, such as frequent urinary urgency, and alleviation of the dysfunctions, such as increased quantity of residual urine. Moreover, it was observed that the enlargement of the prostrate, one of the essential causes of BPH was reduced during a mono-therapy with mono-substances in accordance with the invention, such as leflunomide. This surprising observation is of much relevance as, until present, no active agent has been capable to positively affect symptomatic BPH as well as the functional disorders. This is mainly due to the fact that the active agents and combinations of active agents available lead to a reduction of the enlarged prostate in very few exceptional cases only. However, if the prostate enlargement continues, the obstruction of the flow of urine together with the secondary damages will continue to exist.

A new point of application for the therapy of the invention are factors which play an important role in inflammatory processes. Though it has been known for a long time that BPH is regularly accompanied by inflammatory processes, their role in the further progress in the disease is unclear. The main characteristic of this inflammatory change is the absence of bacterial or viral pathogens. Recent studies support the hypothesis that, in the case of BPH, the inflammatory changes are a consequence of misdirected and excessive immunoreactions. Certain components of the prostate secretion which attract immunoreactive CD4+ T-cells might be the cause. The establishment of reactive T-cells and the setting free of stimulating cytokines by cells of the connective tissue increases the reactivity of T-cells. These set free cytokines such as IFN-gamma, IL-2 and TGF-beta. These cytokines are capable to stimulate the proliferation of smooth muscle cells and epithelial cells. This process is, in many aspects, similar to the process of cicatrisation. Here also, cytokines set free stimulate tissue growth. In contrast to the process of a scar cicatrizing, the pathologic immunoreaction of BPH and the resulting tissue proliferation persist. An effective therapy focused on this mechanism of the disease has not been known and has not been available. With view to recent research results, however, a causal therapy should prevent misdirected and excessive pathological immunoreactions in prostate tissue.

The surprisingly found efficacy of the substances of the invention, in particular of leflunomide, underlines the significance of the inflammatory processes for the genesis and progression of BPH. Thus, due to the surprisingly found efficacy of the substances according to the invention, in particular leflunomide, it was possible to establish a new highly effective and causally acting way of therapy. In comparison to the pharmaceuticals available up to now, leflunomide is characterized by its particularly high efficacy and good application safety. A further feature surprisingly found is the fact that the substances of the invention, e.g. leflunomide, do not have to be combined with another active agent to develop their therapeutic effect. The particularly high efficacy results from the different therapeutic effects. The reduction of the volume of the enlarged prostate is central. It is observed after 2 to 4 months of therapy. With leflunomide treated patients, the reduction of volume can be established by ultrasonography. In comparison with the initial diagnostic findings prior to therapy, a volume reduction of up to 35% with respect to the maximum diameter prior to therapy can be observed with the patient treated. An amelioration of the symptomatic conditions of the patients can already be observed before the volume reduction sets in. Already 4 weeks after starting the leflunomide therapy, the patients can report on a definite abatement of symptomatic conditions. Micturition frequency is lessened, urgency is decreased, during the night phase, the patients have to get up less frequently to pass urine. Moreover, leflunomide treated patients can report that their urinary stream is less frequently disrupted, which also points to an amelioration of the disturbed flow of urine.

The particular safety of the surprisingly observed efficacy of the substances according to the invention, in particular leflunomide, results from the fact that these substances, such as leflunomide, do not have to be combined with other active agents to become effective. Substance combinations always represent a higher treatment risk. On the one hand, this risk is due to the undesired effect of each individual substance. On the other hand, certain undesired effects of different substances can be increased. This applies primarily to the already known substance combination of alplla-blockers and inflammation inhibiting substances which might have noxious and undesired effects on the gastrointestinal tract. Diarrhoea which can already be observed very frequently with a continuous leflunomide treatment is even increased by alpha-blockers. Since the concurrent effect is particularly critical and not predictable by the physician, it is highly advantageous to apply a mono-therapy. A further disadvantage of a combination of substances lies in the fact that the patient dose of the different substances would have to be fine-tuned for each individual patient in order to obtain an optimum effect. This disadvantage is not observed with a treatment of BPH in form of a leflunomide mono-therapy.

In order to achieve therapeutic efficacy of leflunomide, it is preferred to administer single daily oral doses of 20 to 25 mg/d. These are the substance doses which, normally, are also used in the treatment of rheumatoid arthritis. Surprisingly, during treatment of BPH, it was also possible to show that a single daily oral dose of only 10 mg/d also produces a therapeutic effect, Thus, according to the invention, single daily doses of 10 to 30 mg/d are recommended.

### Example:

A male patient who, typically, is 60 to 70 years old. This patient develops rheumatoid arthritis which is progressive in course and requires treatment. Different non-steroidal antiphlogistics can be used in the treatment. In addition to rheumatoid arthritis, the patient develops BPH. In order to treat the rheumatoid arthritis, the patient can be treated with leflunomide in a dosage of 20 mg in form of a daily single dose. Already 4 weeks after starting the treatment, an amelioration of the BPH symptoms can be observed. The success of a BPH therapy is normally assured by anamnestic inquiry, examinations based on imaging techniques and functional examinations. The patient reports on decreased micturition frequency, less frequent passing of urine during the night and the urinary stream being less frequently disrupted. An ultrasonography carried out thereupon establishes a reduced residual urine quantity in comparison with the initial examinations prior to the leflunomide treatment. With the leflunomide treated patient, the quantity of residual urine in the bladder reduced by about 35% can be observed in comparison with the examination prior to start of treatment with leflunomide. The ultrasonograpy of the prostate also provides reliable indications as to a reduction of the size of the prostate.

## Claims

1. Immunomodulating and immunosuppressive active agents as mono-substance for the treatment of BPH and/or non-bacterial prostatitis.

2. Immunomodulating and immunosuppressive active agents according to claim 1, whereby these are selected from the group comprising adalimumab, azathioprine, chloroquine and hydroxychloroquine (anti-malaria agents), cyclosporine (cyclosporine A), D-penicillamine, etanercept, gold salts (e.g. sodium aurothiomalate, auranofine), infliximab, leflunomide, methotrexate (MTX), minocycline (a tretracycline antibiotic), sulfasalazine (SSZ), FK 506, glucocorticoids, SEGRA inhibitors, fingolimod (FTY-720), laquinimod, teriflunomid (HMR-1726), MBP-8298, daclizumab, MLN-1202, TV-5010, BG-12, ZK 117137, temsirolimus, sirolimus, tacrolimus (mitoxantron), basaliximab, MBP-8298, MLN-3897, CCX-915, MK0812, AZD-9056, BMS-345541 and bortezomib.

3. Leflunomide as mono-substance for the treatment of BPH and/or non-bacterial protatitis.

4. Pharmaceutical composition, comprising immunomodulatory and immunosuppressive active agents for the treatment of BPH and/or non-bacterial prostatitis.

5. Pharmaceutical composition according to claim 4, wherein the immunomodulatory and immunosuppressive active agents are selected from the group comprising adalimumab, azathioprine, chloroquine and hydroxychloroquine (anti-malaria agents), cyclosporine (cyclosporine A), D-penicillamine, etanercept, gold salts (e.g. sodium aurothiomalate, auranofine), infliximab, leflunomide, methotrexate (MTX), minocycline (a tretracycline antibiotic), sulfasalazine (SSZ), FK 506, glucocorticoids, SEGRA inhibitors, fingolimod (FTY-720), laquinimod, teriflunomid (HMR-1726), MBP-8298, daclizumab, MLN-1202, TV-5010, BG-12, ZK 117137, temsirolimus, sirolimus, tacrolimus (mitoxantron), basaliximab, MBP-8298, MLN-3897, CCX-915, MKOSI2, AZD-9056, BMS-345541 and bortezomib.

6. Pharmaceutical composition according to any one of claims 4 or 5, wherein the active agent is leflunomide.

7. Immunomodulatory and immunosuppressive active agents for the administration as mono-substance prepared for the treatment of BPH and/or non-bacterial prostatitis.
